# EUROPÄISCHE PATENTSCHRIFT

(11) **EP 1 527 036 B1**
(45) Veröffentlichungstag und Bekanntmachung des Hinweises auf die Patenterteilung: **26.03.2008**
(21) Anmeldenummer: 03771068.8
(22) Anmeldetag: 22.07.2003
(51) Int. Cl.: C07C 7/08, B01D 3/40

(54) **KONTINUIERLICHES VERFAHREN ZUR AUFTRENNUNG EINES C sb 4 /sb -SCHNITTES**
CONTINUOUS METHOD FOR SEPARATING A C SB 4 /SB CUT
PROCEDE CONTINUE DE SEPARATION D'UNE COUPE C SB 4 /SB

(30) Priorität: 24.07.2002 DE 10233620
(43) Veröffentlichungstag der Anmeldung: 04.05.2005
(73) Patentinhaber: BASF SE, 67056 Ludwigshafen (DE)
(72) Erfinder: ADRIAN, Till, 67240 Bobenheim-Roxheim (DE); HILL, Thomas, 67051 Ludwigshafen (DE); KINDLER, Klaus, 67376 Harthausen (DE); HEIDA, Bernd, 67158 Ellerstadt (DE)
(74) Vertreter: Isenbruck, Günter
(86) Internationale Anmeldenummer: PCT/EP2003/007991
(87) Internationale Veröffentlichungsnummer: WO 2004/011406

(56) Entgegenhaltungen:
- EP-A- 0 667 329
- DE-A- 10 022 465
- DE-A- 10 105 660
- DE-A- 19 818 810
- US-A- 6 137 023
- US-A1- 2002 087 040

## Beschreibung

Die Erfindung betrifft ein kontinuierliches Verfahren zur Auftrennung eines C₄-Schnittes durch Extraktivdestillation mit einem selektiven Lösungsmittel.

Der Begriff C₄-Schnitt bezeichnet Gemische von Kohlenwasserstoffen mit überwiegend 4 Kohlenstoffatomen pro Molekül. C₄-Schnitte werden beispielsweise bei der Herstellung von Ethylen und/oder Propylen durch thermisches Spalten, üblicherweise in Steamcrackern oder FCC-Crackern (Fluidized Catalytic Cracking) einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an sonstigen Kohlenwasserstoffen, darunter Butine, insbesondere 1-Butin (Ethylacetylen) und Butenin (Vinylacetylen). Dabei beträgt der 1,3-Butadiengehalt von C₄-Schnitten aus Steamcrackern im allgemeinen 10 bis 80 Gew.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Die Auftrennung von C₄-Schnitten ist wegen der geringen Unterschiede in den relativen Flüchtigkeiten der Komponenten ein kompliziertes destillationstechnisches Problem. Daher wird die Auftrennung durch eine sogenannte Extraktivdestillation durchgeführt, d.h. eine Destillation unter Zugabe eines selektiven Lösungsmittels (auch als Extraktionsmittel bezeichnet), das einen höheren Siedepunkt als das aufzutrennende Gemisch aufweist und die Unterschiede in den relativen Flüchtigkeiten der aufzutrennenden Komponenten erhöht.

Es sind eine Vielzahl von Verfahren zur Auftrennung von C₄-Schnitten mittels Extraktivdestillation unter Verwendung von selektiven Lösungsmitteln bekannt. Ihnen ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, häufig im Bereich von 20 bis 80°C und bei moderaten Drücken, häufig bei Normaldruck bis 6 bar, das selektive Lösungsmittel mit den Komponenten aus dem C₄-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt in einem oder mehreren weiteren Verfahrensschritten die Komponenten fraktioniert aus dem selektiven Lösungsmittel, freigesetzt.

Häufig wird die Extraktivdestillation von C₄-Schnitten in der Weise gefahren, dass die Komponenten des C₄-Schnittes, für die das selektive Lösungsmittel eine geringere Affinität als für 1,3-Butadien hat, insbesondere die Butane und die Butene, im Wesentlichen in der Gasphase verbleiben, wogegen 1,3-Butadien sowie weitere Kohlenwasserstoffe, für die das selektive Lösungsmittel eine höhere Affinität als für 1,3-Butadien hat, vom selektiven Lösungsmittel im Wesentlichen vollständig absorbiert werden. Die Gasphase wird als Kopfstrom abgezogen und häufig als Raffinat 1 bezeichnet. Ein derartiges Verfahren ist beispielsweise in der DE-A 198 188 10 beschrieben, wobei das Raffinat 1 der in den Figuren 1 und 2 mit Gbc bezeichnete Kopfstrom der Extraktivdestillationskolonne E I ist.

Für die weitere Verwertung des Raffinats 1 ist es jedoch in der Regel wirtschaftlicher, wenn die Butane und die Butene jeweils als getrennte Ströme vorliegen. Die in den nachfolgenden Stufen für die Weiterverarbeitung der Butene eingesetzten Apparate werden dadurch kleiner und Butane können direkt als wertvoller Cracker-Feed gewonnen werden.

Daher schlägt die DE-A 102 193 75 ein Verfahren zur Auftrennung eines C₄-Schnittes durch Extraktivdestillation vor, wonach Butane und Butene in jeweils getrennten Strömen erhältlich sind. Hierfür sind jedoch zwei Verfahrensstufen erforderlich, wobei in einer ersten Verfahrensstufe I in einer Waschzone ein die Butane enthaltender Kopfstrom und in einer zweiten Verfahrensstufe II in einer Ausgaserzone ein die Butene enthaltender Kopfstrom abgezogen wird.

Demgegenüber war es Aufgabe der Erfindung, ein verbessertes, insbesondere wirtschaftlicheres, mit geringerem Energieverbrauch durchführbares Verfahren zur Auftrennung eines C₄-Schnittes in 1,3-Butadien, Butene und Butane als jeweils getrennte Ströme durch Extraktivdestillation zur Verfügung zu stellen.

Die Aufgabe wird durch ein kontinuierliches Verfahren zur Auftrennung eines C₄-Schnittes durch Extraktivdestillation mit einem selektiven Lösungsmittel in einer Extraktivdestillationskolonne gelöst, das dadurch gekennzeichnet ist, dass in der Extraktivdestillationskolonne in Längsrichtung derselben eine Trennwand unter Ausbildung eines ersten Teilbereichs, eines zweiten Teilbereichs und eines unteren gemeinsamen Kolonnenbereiches angeordnet ist, und dass aus dem ersten Teilbereich ein die Butane umfassender Kopfstrom, aus dem zweiten Teilbereich ein die Butene umfassender Kopfstrom und aus dem unteren gemeinsamen Kolonnenbereich ein die Kohlenwasserstoffe aus dem C₄-Schnitt umfassender Strom abgetrennt wird, die im selektiven Lösungsmittel besser löslich als die Butane und die Butene sind.

Durch die Erfindung wird somit ein Verfahren zur Auftrennung eines C₄-Schnittes durch Extraktivdestillation zur Verfügung gestellt, wonach die Abtrennung von Butanen und Butenen als jeweils getrennte Kopfströme einer einzigen Extraktivdestillationskolonne ermöglicht wird.

Erfindungsgemäß wird die Extraktivdestillation in einer Trennwandkolonne durchgeführt, in der eine Trennwand unter Ausbildung eines ersten Teilbereichs, eines zweiten Teilbereichs und eines unteren gemeinsamen Kolonnenbereiches angeordnet ist.

Trennwandkolonnen werden in bekannter Weise für komplexere Trennaufgaben, in der Regel für Gemische von mindestens drei Komponenten, wobei die Einzelkomponenten jeweils in reiner Form erhalten werden sollen, eingesetzt. Sie weisen eine Trennwand auf, das heißt ein in der Regel in Kolonnenlängsrichtung ausgerichtetes ebenes Blech, das eine Quervermischung der Flüssigkeits- und Brüdenströme in Teilbereichen der Kolonne unterbindet.

Vorliegend wird eine besonders ausgestaltete Trennwandkolonne eingesetzt, deren Trennwand bis zum obersten Punkt der Kolonne durchgezogen ist und die somit eine Vermischung von Flüssigkeits- und Brüdenströmen lediglich im unteren gemeinsamen Kolonnenbereich gestattet. Der sogenannte erste und zweite Teilbereich sind durch die Trennwand voneinander getrennt.

Je nach Zusammensetzung des der Extraktivdestillationskolonne zugeführten C₄-Schnittes sowie der vorgegebenen Spezifikationen für die in der Extraktivdestillationskolonne abzutrennenden Fraktionen kann die Länge der Trennwand sowie die horizontale Lage derselben in der Extraktivdestillationskolonne unterschiedlich ausgestaltet sein. So ist es beispielsweise möglich, die Trennwand mittig oder außermittig anzuordnen.

Erfindungsgemäß wird aus dem ersten Teilbereich der als Trennwandkolonne ausgebildeten Extraktivdestillationskolonne ein die Butane umfassender Kopfstrom und aus dem zweiten Teilbereich ein die Butene umfassender Kopfstrom abgezogen. Aus dem unteren gemeinsamen Kolonnenbereich wird ein Strom abgezogen, der die Kohlenwasserstoffe aus dem C₄-Schnitt umfasst, der im selektiven Lösungsmittel besser löslich sind als die Butane und die Butene.

Für die vorliegende Trennaufgabe sind selektive Lösungsmittel geeignet, deren Affinität zu Kohlenwasserstoffen mit Einfachbindungen in Richtung zu Kohlenwasserstoffen mit Doppelbindungen und weiter zu konjugierten Doppelbindungen und Dreifachbindungen zunimmt, bevorzugt dipolare, besonderes bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon, im Folgenden abgekürzt als NMP bezeichnet. Im allgemeinen werden alkylsubstituierte niedere aliphatische Säureamide oder N-alkylsubstituierte cyclische Säureamide verwendet. Besonders vorteilhaft sind Dimethylformamid, Acetonitril, Furfurol und insbesondere NMP.

Es können jedoch auch Mischungen dieser Lösungsmittel untereinander, zum Beispiel von NMP mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist NMP, bevorzugt in wässriger Lösung, vorteilhaft mit 0 bis 20 Gew. -% Wasser, insbesondere mit 7 bis 10 Gew.-% Wasser, besonders bevorzugt mit 8,3 Gew.-% Wasser.
Als C₄-Schnitt für den Einsatz im vorliegenden Verfahren kann vorteilhaft ein Gemisch von Kohlenwasserstoffen eingesetzt werden, das durch thermisches Spalten einer Petroleumfraktion erhalten wurde. Ein derartiges Gemisch weist typischerweise Zusammensetzungen in Gew.-% in den nachstehenden Bereichen auf:

| | |
|---|---|
| 1,3-Butadien | 10 bis 80 |
| Butene | 10 bis 60 |
| Butane | 5 bis 40 |
| sonstige C₄-Kohlenwasserstoffe und sonstige Kohlenwasserstoffe, insbesondere | 0,1 bis 5 |
| C₃- und C₅-Kohlenwasserstoffe | 0 bis maximal 5. |

Die Erfindung ist jedoch nicht eingeschränkt bezüglich der einsetzbaren C₄-Schnitte. Beispielsweise sind auch C₄-Schnitte aus sogenannten FCC-Crackern (Fluidized Catalytic Cracking) einsetzbar, in der Regel eine Zusammensetzung von 20 bis 70 Gew.-% an Butanen, 30 bis 80 Gew.- an Butenen, Rest sonstige C₃- bis C₅-Kohlenwasserstoffe aufweisen.

Der C₄-Schnitt wird der Extraktivdestillationskolonne, bevorzugt dem durch die Trennwand abgetrennten ersten Teilbereich derselben dampfförmig oder flüssig zugeführt. Besonders bevorzugt wird der C₄-Schnitt etwa dem mittleren Bereich des ersten Teilbereichs der Extraktivdestillationskolonne zugeführt.

Das selektive Lösungsmittel wird als flüssiger Strom auf beide Teilbereiche der Extraktivdestillationskolonne, jeweils in deren oberem Teil, aufgegeben.

Bevorzugt wird ein die Butane umfassender Strom als Kopfstrom aus dem ersten Teilbereich der Extraktivdestillationskolonne dampfförmig abgezogen, in einem Kondensator am Kolonnenkopf kondensiert, teilweise als Rücklauf wieder auf den ersten Teilbereich aufgebeben, und im Übrigen abgezogen.

Entsprechend wird aus dem zweiten Teilbereich der Extraktivdestillationskolonne ein die Butene umfassender Kopfstrom abgezogen, der bevorzugt in einem Kondensator kondensiert, teilweise als Rücklauf wieder auf den zweiten Teilbereich aufgegeben und im Übrigen abgezogen wird.

Die vorstehend verwendete Formulierung, dass der Kopfstrom aus dem Teilbereich der Extraktivdestillationskolonne jeweils Butane bzw. Butene umfasst, bedeutet, dass die jeweiligen Ströme, je nach geforderter Spezifikation, überwiegend Butane bzw. Butene umfassen, das heißt in der Regel mindestens 80 Gew.-% Butane bzw. Butene, vorzugsweise 95 bis 99 Gew.-% Butane bzw. Butene. In Einzelfällen können auch Spezifikationen mit Reinheiten oberhalb von 99 Gew.-% Butane bzw. Butene gefordert sein. Die weiteren Komponenten der jeweiligen Kopfströme sind insbesondere Butane im Butenestrom und umgekehrt sowie Spuren weiterer Kohlenwasserstoffe.

Erfindungsgemäß wird aus dem unteren gemeinsamen Kolonnenbereich der Extraktivdestillationskolonne ein Strom abgetrennt, der die Kohlenwasserstoffe aus dem C₄-Schnitt umfasst, die im selektiven Lösungsmittel besser löslich sind als die Butane und die Butene.

Hierbei ist es bevorzugt möglich, den Strom, der die Kohlenwasserstoffe aus dem C₄-Schnitt umfasst, die im selektiven Lösungsmittel besser löslich sind als die Butane und die Butene als Seitenstrom aus dem unteren gemeinsamen Kolonnenbereich und das selektive Lösungsmittel als Sumpfstrom abzuziehen. In dieser Verfahrensführung ist somit im unteren Teil des unteren gemeinsamen Kolonnenbereichs der Extraktivdestillationskolonne ein Desorptionsschritt zur Abtrennung der Kohlenwasserstoffe aus dem damit beladenen Lösungsmittel mit integriert. Es ist jedoch gleichermaßen möglich, den Desorptions-Verfahrensschritt in einem von der Extraktivdestillationskolonne getrennten Apparat durchzuführen, das heißt zunächst aus der Extraktivdestillationskolonne als Sumpfstrom die Kohlenwasserstoffe aus dem C₄-Schnitt, die im selektiven Lösungsmittel besser löslich sind als die Butane und die Butene gemeinsam mit dem selektiven Lösungsmittel als Sumpfstrom abzutrennen.

Der Ausgestaltung der Extraktivdestillationskolonne als Trennwandkolonne ist eine Anordnung von zwei oder mehreren, insbesondere von zwei oder drei thermisch gekoppelten Kolonnen äquivalent. Der Energiebedarf ist hierbei der entsprechenden Trennwandkolonne vergleichbar. Die Erfindung umfasst somit auch sämtliche apparativen Varianten, wonach die Extraktivdestillationskolonne nicht als Trennwandkolonne, sondern als zwei oder mehrere, insbesondere als zwei oder drei thermisch gekoppelte Kolonnen ausgebildet ist.

Die Erfindung ist nicht eingeschränkt bezüglich der in der Extraktivdestillationskolonne einsetzbaren trennwirksamen Einbauten.

Die Anzahl der theoretischen Trennstufen im Bereich der Trennwand ist insbesondere von der Zusammensetzung des zugeführten C₄-Schnittes, dem eingesetzten Lösungsmittel, sowie von den geforderten Spezifikationen für den die Butane und den die Butene umfassenden Kopfstrom abhängig. Bevorzugt können in der Extraktivdestillationskolonne im Bereich der Trennwand 10 bis 80 theoretische Trennstufen, insbesondere 25 theoretische Trennstufen angeordnet sein.

Die Zusammensetzung des zugeführten C₄-Schnittes, das eingesetzte Lösungsmittel sowie die geforderten Spezifikationen für den die Butane sowie für den die Butene umfassenden Kopfstrom sind auch maßgeblich für die Wahl des Zulaufbodens für den C₄-Schnitt, bevorzugt im ersten Teilbereich der Extraktivdestillationskolonne sowie für die Zulaufmengen des im oberen Teil des ersten sowie des zweiten Teilbereichs aufgegebenen selektiven Lösungsmittels.

Vorteilhaft ist am Kopf der Extraktivdestillationskolonne, in jedem der Teilbereiche derselben, jeweils ein Kondensator für die Brüdenströme angeordnet.

Es ist vorteilhaft möglich, in der Extraktivdestillationskolonne zusätzlich eine Selektivhydrierung in heterogener Katalyse mit Wasserstoff der Dreifachbindungen aufweisenden Kohlenwasserstoffe aus dem C₄-Schnitt zu Doppelbindungen aufweisenden Kohlenwasserstoffen durchzuführen. Hierfür ist es erforderlich, geeignete Einbauten in der Extraktivdestillationskolonne vorzusehen, die mit heterogenen Katalysatoren bestückt sind sowie einen Wasserstoffstrom in die Kolonne einzuleiten, bevorzugt unterhalb der Zuführung des C₄-Schnittes, in den unteren gemeinsamen Kolonnenbereich der Extraktivdestillationskolonne.

Der im vorliegenden Verfahren die Butene, das heißt 1-Buten, 2-Butene (cis und trans) und Isobuten umfassende Strom kann in unterschiedlichen Verfahren weiterverarbeitet werden.

Der in den nachfolgenden Ausführungen in Verbindung mit der Kennzeichnung von Strömen verwendete Begriff "überwiegend umfassend" bedeutet, dass die Ströme mindestens 60 Gew.-%, vorzugsweise mindestens 80 Gew.-%, besonders bevorzugt mindestens 95 Gew.-% der jeweils angegebenen Hauptkomponente enthalten.

Vorteilhaft kann die Weiterverarbeitung zu Isobuten als einzigem Wertprodukt oder zusätzlich zu einem 1-Buten oder einem 2-Butene umfassenden Wertprodukt erfolgen.

Hierfür ist es in einer ersten Verfahrensvariante möglich, die Weiterverarbeitung in einer Reaktivdestillationskolonne zu einem überwiegend Isobuten umfassenden Strom und einem überwiegend 2-Butene umfassenden Strom durchzuführen, wobei in der Reaktivdestillationskolonne 1-Buten- zu 2-Butenen hydroisomerisiert wird und der überwiegend Isobuten umfassende Strom als Kopfstrom der Reaktivdestillationskolonne und der überwiegend 2-Butene umfassende Strom als Sumpfstrom der Reaktivdestillationskolonne abgezogen wird.

In einer weiteren Verfahrensvariante wird der die Butene umfassende Strom einer selektiven Veretherung des Isobutens und Auftrennung in einen den Isobutenether umfassenden Strom und in einen 1-Buten und 2-Butene umfassenden Strom unterworfen wird und der 1-Buten und die 2-Butene umfassende Strom anschließend durch Gasphasenisomerisierung der 2-Butene zu einem überwiegend 1-Buten umfassenden Strom oder durch Hydroisomerisierung des 1-Butens zu einem überwiegend 2-Butene umfassenden Strom weiterverarbeitet.

Es ist auch möglich, die Weiterverarbeitung durch Skelettisomerisierung von 1-Buten und 2-Butenen zu Isobutenen durchzuführen, wobei ein überwiegend Isobuten umfassender Strom erhalten wird.

Die Weiterverarbeitung des nach dem vorliegenden Verfahren erhaltenen, Butene umfassenden Stromes ist auch in der Weise möglich, dass kein Isobuten als Wertprodukt angestrebt, wird sondern dass die Weiterverarbeitung durch eine der nachfolgend aufgeführten Verfahrensvarianten durchgeführt wird:
In einer Verfahrensvariante wird Isobuten abgetrennt und durch Skelettisomerisierung zu einem überwiegend 1-Buten und 2-Butene umfassenden Strom aufgearbeitet.
In einer weiteren Variante wird Isobuten abgetrennt und durch Hydrierung zu einem überwiegend Isobutan umfassenden Strom weiterverarbeitet, der vorzugsweise einem Cracker zugeführt oder durch Skelettisomerisierung zu einem überwiegend n-Butan umfassenden Strom und Dehydrierung desselben zu einem überwiegend 1-Buten und 2-Butene umfassenden Strom weiterverarbeitet wird.
Es ist auch möglich, aus dem die Butene umfassenden Strom das Isobuten selektiv zu den entsprechenden C₈-Kohlenwasserstoffen zu dimerisieren. Die C₈-Kohlenwaserstoffe können anschließend in einer einfachen Destillation von einem 1-Buten und 2-Butene umfassenden Strom abgetrennt werden.
In einer bevorzugten Verfahrensvariante wird der aus der Extraktivdestillationskolonne abgezogene Strom, der die im selektiven Lösungsmittel besser als die Butane und die Butene löslichen Kohlenwasserstoffe umfasst, destillativ weiter aufgearbeitet. Dabei wird der aus der Extraktivdestillationskolonne abgezogene Strom, der die im selektiven Lösungsmittel besser als die Butane und Butene löslichen Kohlenwasserstoffe umfasst, einer ersten Destillationskolonne zugeführt, worin er in einen Kopfstrom aufgetrennt wird, umfassend 1,3-Butadien, Propin, gegebenenfalls weitere Leichtsieder und gegebenenfalls Wasser, sowie in einen Sumpfstrom, umfassend 1,3-Butadien, 1,2-Butadien, Acetylene sowie gegebenenfalls weitere Schwersieder, wobei der Anteil des 1,3-Butadiens im Sumpfstrom der Destillationskolonne dergestalt geregelt wird, dass er mindestens so hoch ist, dass er die Acetylene außerhalb des selbst zersetzungsgefährdeten Bereiches verdünnt. Der Kopfstrom der ersten Destillationskolonne wird einer zweiten Destillationskolonne zugeführt und in der zweiten Destillationskolonne in einen Kopfstrom, umfassend Propin, gegebenenfalls weitere Leichtersieder und gegebenenfalls Wasser und in einen Sumpfstrom, umfassend Rein-1,3-Butadien, aufgetrennt.

Der der destillativen Aufarbeitung zugeführte Strom umfasst überwiegend 1,3-Butadien und wird daher als Roh-1,3-Butadien-Strom bezeichnet.

Die Zusammensetzung des Roh-1,3-Butadienstromes ist abhängig von der Zusammensetzung des C₄-Schnittes, der der Extraktivdestillation zugeführt wurde und umfasst in der Regel die gesamten Acetylene, das gesamte 1,2-Butadien, 30 bis 70 % des cis-2-Butens sowie mindestens 99 % des 1,3-Butadiens aus dem C₄-Schnitt.

Dabei werden vorliegend die niedriger als 1,3-Butadien siedenden Kohlenwasserstoffe als Leichtsieder und die höher als 1,3-Butadien siedenden Kohlenwasserstoffe als Schwersieder bezeichnet. Ein typischer Leichtsieder ist Propin, Schwersieder sind überwiegend Kohlenwasserstoffe mit einer Dreifachbindung, im Folgenden als Acetylene bezeichnet, insbesondere 1-Butin (Ethylacetylen) und Butenin (Vinylacetylen).

Der vorliegend in Verbindung mit der Zusammensetzung von bei der destillativen Aufarbeitung erhaltenen Strömen verwendete Begriff "gegebenenfalls" bedeutet, dass die danach aufgeführten Komponenten, je nach konkreter Verfahrensführung, insbesondere je nach Zusammensetzung des eingesetzten C₄-Schnittes, des eingesetzten Lösungsmittels und/oder eingesetzter Hilfsstoffe in den jeweiligen Strömen vorhanden sein können.

Die destillative Abtrennung der Acetylene und des 1,2-Butadiens aus dem Roh-1,3-Butadien ist aufgrund der hohen Reaktivität derselben sowie aufgrund der geringen Unterschiede in relativen Flüchtigkeiten der den Roh-1,3-Butadien-Strom bildenden Komponenten ein kompliziertes destillationstechnisches Problem. Es wurde jedoch überraschend gefunden, dass die destillative Abtrennung der Acetylene und des 1,2-Butadiens mit vertretbarem energetischem Aufwand möglich ist und dabei gleichzeitig eine sichere Verfahrensführung gewährleistet werden kann, indem die Acetylene und das 1,2-Butadien als Sumpfstrom aus einer Destillationskolonne abgezogen werden, und dabei mit 1,3-Butadien außerhalb des selbstzersetzungsgefährdeten Bereiches verdünnt werden. Hierfür ist in der Regel eine Verdünnung des Sumpfstromes auf unterhalb von 30 Mol-%, Acetylene ausreichend.

Nach einer bevorzugten Verfahrensführung wird somit der aus der Extraktivdestillationskolonne oder einer nachgeschalteten Desorptionskolonne abgezogene Roh-1,3-Butadien-Strom in einer Destillationskolonne einer bezüglich 1,3-Butadien unscharfen destillativen Auftrennung unterworfen, wobei die Acetylene als Sumpfstrom abgezogen werden, der mit 1,3-Butadien außerhalb des selbstzersetzungsgefährdeten Bereiches verdünnt ist. Im Übrigen wird Butadien, gemeinsam mit Propin und gegebenenfalls weiteren Leichtsiedern, als Kopfstrom der Destillationskolonne abgezogen.

Der Kopfstrom der Destillationskolonne wird bevorzugt in einem Kondensator am Kolonnenkopf kondensiert, teilweise als Rücklauf erneut auf die Kolonne aufgegeben und im Übrigen einer zweiten Destillationskolonne zugeführt, und darin in einen Kopfstrom aufgetrennt, der Propin und gegebenenfalls weitere Leichtsieder umfasst und in einen Sumpfstrom, umfassend Rein-1,3-Butadien.

In beiden vorstehend beschriebenen Destillationskolonnen können grundsätzlich alle für Butadien-Destillationen üblichen trennwirksamen Einbauten eingesetzt werden. Aufgrund ihrer geringeren Verschmutzungsneigung sind Böden besonders geeignet.

Der Begriff Rein-1,3-Butadien bezeichnet vorliegend einen Strom mit einem Gehalt von mindestens 99 Gew.-% 1,3-Butadien, bevorzugt von mindestens 99,6 Gew.-% 1,3-Butadien, Rest Verunreinigungen, insbesondere 1,2-Butadien und cis-2-Buten.

In einer bevorzugten Verfahrensalternative werden der Sumpfstrom aus der ersten Destillationskolonne und der Kopfstrom aus der zweiten Destillationskolonne einer Reakivdestillationskolonne zugeführt, in der in heterogener Katalyse mit Wasserstoff eine Selektivhydrierung der Dreifachbindungen aufweisenden Kohlenwasserstoffe zu Doppelbindungen aufweisenden Kohlenwasserstoffen durchgeführt wird, unter Erhalt eines Kopfstromes, umfassend 1,3-Butadien, Butane, Butene sowie Restmengen an nicht-hydrierten Kohlenwasserstoffen mit Dreifachbindungen und einen Schwersieder umfassenden Sumpfstrom, der ausgeschleust wird.

Insbesondere wird Vinylacetylen zum Wertprodukt 1,3-Butadien selektiv hydriert.

Der Kopfstrom der Reaktivdestillationskolonne kann bevorzugt in die Extraktivdestillationskolonne rezykliert werden. Es ist jedoch auch möglich, den Kopfstrom der Reaktivdestillationskolonne oder einen Teilstrom desselben aus der Anlage abzuziehen, und weiter zu verarbeiten, beispielsweise in einem Cracker, oder zu verbrennen.

Die bevorzugte Verfahrensweise mit der Extraktivdestillation nachgeschalteter Selektivhydrierung der Acetylene ist verfahrenstechnisch insbesondere bezüglich der Auswahlmöglichkeiten für den Katalysator vorteilhaft, da die Selektivhydrierung in einer Verfahrensstufe durchgeführt wird, in der praktisch kein selektives Lösungsmittel mehr im Reaktionsgemisch vorliegt. Würde die Selektivhydrierung dagegen, wie in bekannten Verfahren, in der Extraktivdestillationskolonne und somit in Gegenwart des selektiven Lösungsmittels durchgeführt werden, so wäre die Auswahl des Katalysators erheblich durch das selektive Lösungsmittel eingeschränkt, das die Hydrierung unselektiver machen kann. In der der Extraktivdestillation nachgeschalteten Selektivhydrierung gibt es dagegen keine derartigen Einschränkungen bezüglich der Katalysatorauswahl.

Die Erfindung wir im Folgenden anhand einer Zeichnung sowie von Ausführungsbeispielen näher erläutert.

Es zeigen im Einzelnen:
- Figur 1: die schematische Darstellung einer Extraktivdestillationskolonne EDK mit Trennwand TW, und
- Figur 2: die schematische Darstellung einer bevorzugten Ausführungsform einer Anlage zur Auftrennung eines C₄-Schnittes in einer Extraktivdestillationskolonne mit nachgeschalteter destillativer Auftrennung des Roh-1,3-Butadien-Stromes aus der Extraktivdestillationskolonne in zwei Destillationskolonnen und nachgeschalteter Selektivhydrierung.

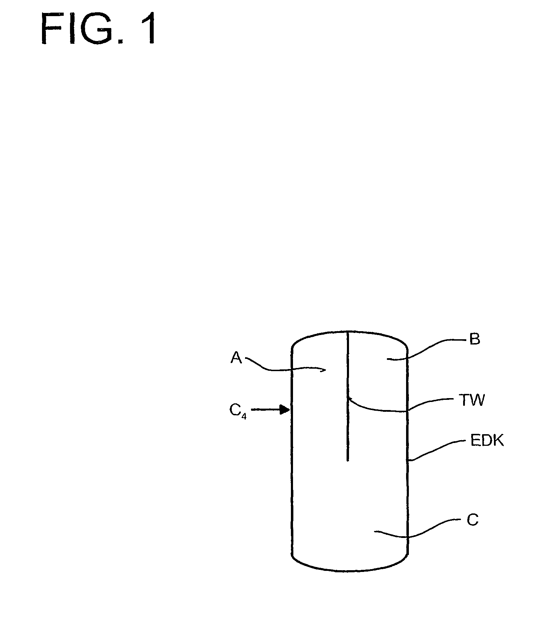

Die in Fig. 1 schematisch dargestellte Extraktivdestillationskolonne EDK weist eine in Längsrichtung derselben angeordnete Trennwand TW auf, die bis zum obersten Punkt der Extraktivdestillationskolonne EDK durchgezogen ist, und dieselbe in einen ersten Teilbereich A, einen zweiten Teilbereich B sowie einen unteren gemeinsamen Kolonnenbereich C auftrennt. Der C₄-Schnitt C₄ wird der Extraktivdestillationskolonne EDK in deren Teilbereich A zugeführt.

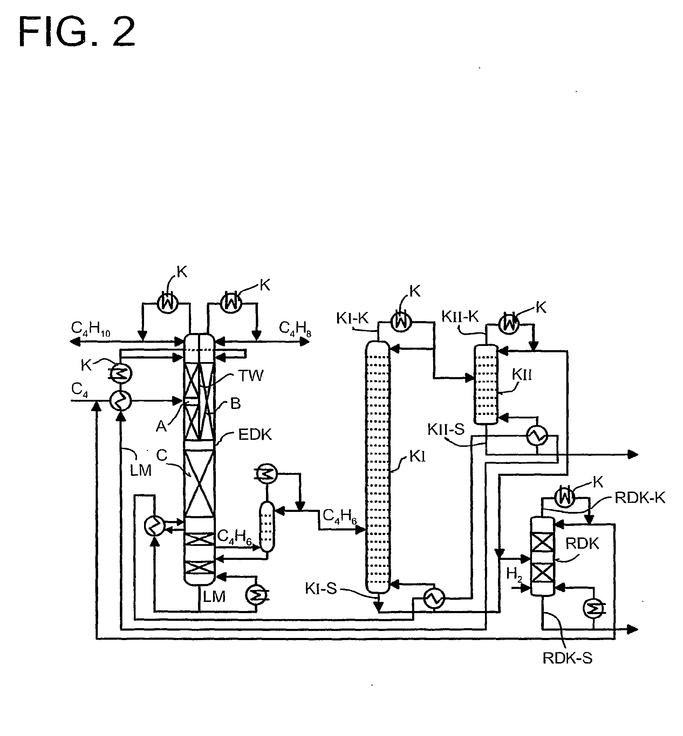

Die in Fig. 2 schematisch dargestellte Anlage zeigt eine Extraktivdestillationskolonne EDK zur Auftrennung eines C₄-Schnittes (C₄), die in ihrem oberen Teil eine in Längsrichtung angeordnete Trennwand TW aufweist, die die Extraktivdestillationskolonne EDK in einen ersten Teilbereich A, einen zweiten Teilbereich B sowie einen unteren gemeinsamen Kolonnenbereich C aufteilt. Der C₄-Schnitt (C₄) der, wie in der Fig. dargestellt, vorteilhaft in einem Wärmetauscher durch Wärmetausch mit dem selektiven Lösungsmittel LM aufgewärmt, insbesondere verdampft werden kann, wird der Extraktivdestillationskolonne EDK im ersten Teilbereich A derselben zugeführt. Auf beide Teilbereiche A und B wird, jeweils von oben, ein flüssiger Strom des selektiven Lösungsmittels LM aufgegeben, das wie in der Fig. dargestellt, vorteilhaft durch Wärmetausch mit dem C₄-Schnitt und anschließend in einem Kondensator abgekühlt wird. Aus dem ersten Teilbereich A der Extraktivdestillationskolonne EDK wird eine die Butane umfassender Kopfstrom C₄H₁₀ abgezogen, in einem Kondensator K kondensiert, teilweise als Rücklauf auf den ersten Teilbereich A der Extraktivdestillationskolonne EDK wieder aufgegeben und im Übrigen abgezogen. Analog wird aus dem zweiten Teilbereich B der Extraktivdestillationskolonne EDK ein die Butene umfassender Kopfstrom C₄H₈ abgezogen, in einem Kondensator K kondensiert, teilweise als Rücklauf wieder auf den zweiten Teilbereich B aufgegeben und im Übrigen abgezogen.

Aus dem unteren gemeinsamen Kolonnenbereich C der Extraktivdestillationskolonne EDK wird ein Strom C₄H₆ abgezogen, der die Kohlenwasserstoffe enthält, die im selektiven Lösungsmittel besser löslich sind als die Butane und die Butene, überwiegend 1,3-Butadien.

Bevorzugt ist es möglich, wie in Fig. 2 dargestellt, den Strom C₄H₆ einer kurzen Seitenkolonne S zuzuführen, in der destillativ ein das Lösungsmittel umfassender Sumpfstrom abgezogen und der Extraktivdestillationskolonne EDK erneut zugeführt wird. Die kurze Seitenkolonne, deren Einsatz nicht zwingend ist, dient somit der Rückgewinnung von Lösungsmittelspuren aus dem Roh-1,3-Butadienstrom.

Aus dem Sumpf der Extraktivdestillationskolonne EDK wird ein überwiegend das selektive Lösungsmittel LM umfassender Strom abgezogen. Die Wärme des Lösungsmittels wird, je nach konkreten Standortbedingungen der Anlage, insbesondere Verfügbarkeit von Kühlmitteln, Einbindung in andere Anlagen bzw. Weiterverarbeitungsketten, über unterschiedliche Wärmetauscher abgegeben und der abgekühlte Lösungsmittelstrom schließlich in die Extraktivdestillationskolonne EDK, in deren oberen Bereich, rezykliert.

Der Seitenstrom der Extraktivdestillationskolonne EDK, Strom C₄H₆, vorliegend als Roh-1,3-Butadien-Strom bezeichnet, wird einer ersten Destillationskolonne K I zugeführt, und in derselben in einen Kopfstrom K I-K und einen Sumpfstrom K I-S aufgetrennt. Der Kopfstrom K I-K wird in einem Kondensator K am Kolonnenkopf kondensiert, teilweise als Rücklauf wieder auf die Kolonne aufgegeben, und im Übrigen abgezogen und einer zweiten Destillationskolonne K II zugeführt. Der Sumpfstrom K I-S wird abgezogen und einer Reaktivdestillationskolonne RDK zugeführt.

In der zweiten Destillationskolonne K II erfolgt eine Auftrennung in einen Kopfstrom K II-K, der in einem Kondensator K kondensiert, teilweise als Rücklauf wieder auf die Kolonne aufgegeben und im Übrigen ebenfalls in die Reaktivdestillationskolonne RDK geführt wird. Der Sumpfstrom K II-S der zweiten Destillationskolonne K II wird als Rein-1,3-Butadien-Strom abgezogen.

In der Reaktivdestillationskolonne RDK werden durch Selektivhydrierung mit Wasserstoff in Gegenwart eines heterogenen Katalysators die Dreifachbindungen aufweisenden Kohlenwasserstoffe zu Doppelbindungen aufweisenden Kohlenwasserstoffen hydriert. Es wird ein Kopfstrom RDK-K abgezogen, in einem Kondensator K kondensiert, teilweise erneut auf der Reaktivdestillationskolonne RDK aufgegeben und im Übrigen vorzugsweise, wie in der Fig. dargestellt, in die Extraktivdestillationskolonne EDK rezykliert.

Der Sumpfstrom der Reaktivdestillationskolonne, Strom RDK-S, der überwiegend Schwersieder enthält, wird aus der Anlage ausgeschleust und vorzugsweise verbrannt.

### Beispiel: Extraktivdestillation

Ein C₄-Schnitt aus einem Steam-Cracker mit der nachstehend in Gew.-% aufgeführten Zusammensetzung wurde einer Extraktivdestillationskolonne., die als Trennwandkolonne mit bis zum obersten Punkt der Kolonne durchgezogener Trennwand ausgebildet war, und die insgesamt 80 theoretische Trennstufen, davon 25 im Bereich der Trennwand aufwies, auf der 68 theoretischen Trennstufe, bei Zählung der Trennstufen von unten, dem in der Zeichnung mit A bezeichneten ersten Teilbereich zugeführt. Als selektives Lösungsmittel wurde NMP mit 8,3 Gew.-% Wasser eingesetzt.

| | |
|---|---|
| Propen | 0,02 |
| Propadien | 0,04 |
| Propin | 0,06 |
| n-Butan | 5,74 |
| i-Butan | 2,44 |
| n-Buten | 13,88 |
| i-Buten | 25,63 |
| t-Buten-2 | 4,44 |
| cis-Buten-2 | 2,95 |
| 1,3-Butadien | 43,81 |
| 1,2-Butadien | 0,14 |
| 1-Butin | 0,12 |
| Vinylacetylen | 0,73. |

Aus dem ersten Teilbereich A der Extraktivdestillationskolonne EDK wurde ein überwiegend Butane umfassender Kopfstrom abgezogen, der die folgende Zusammensetzung in Gew.-% aufwies:

| | |
|---|---|
| Propen | 0,19 |
| n-Butan | 62,02 |
| i-Butan | 27,98 |
| n-Buten | 6,63 |
| i-Buten | 2,71 |
| trans-Buten-2 | 0,24 |
| H₂O | 0,23 |

Aus dem Teilbereich B der Extraktivdestillationskolonne wurde ein überwiegend Butene umfassender Kopfstrom mit folgender Zusammensetzung in Gew.-% abgezogen:

| | |
|---|---|
| Propadien | 0,07 |
| n-Butan | 0,91 |
| i-Butan | 0,10 |
| n-Buten | 28,57 |
| i-Buten | 54,55 |
| trans-Buten-2 | 9,48 |
| cis-Buten-2 | 6,32 |

Gegenüber einem bekannten Verfahren mit gemeinsamer Abtrennung von Butanen und Butenen in einer Extraktivdestillationskolonne und nachfolgender ButaneButene-Auftrennung in einem zusätzlichen Apparat wurde eine Energieeinsparung von ca. 20 % erreicht.

### Beispiel: Destillative Aufarbeitung von Roh-1,3-Butadien

Ein Roh-1,3-Butadienstrom, der entsprechend dem vorstehend aufgeführten Beispiel Extraktivdestillation aus einem dort aufgeführten C₄-Schnitt erhalten wurde, wurde einer Destillationskolonne mit 80 theoretischen Trennstufen auf der 25. Stufe, bei Zählung der Trennstufen von unten nach oben, zugeführt. Der Roh-1,3-Butadienstrom C₄H₆ hatte die folgende Zusammensetzung in Gew.-%:

| | |
|---|---|
| Propin | 0.11 |
| 1,3-Butadien | 98,58 |
| 1,2-Butadien | 0,30 |
| 1-Butin | 0,30 |
| Vinylacetylen | 0,56 |
| Wasser | 0,15. |

Er wurde in der Destillationskolonne K I in einen Kopfstrom K I-K aufgetrennt, mit nachstehender Zusammensetzung in Gew.-%:

| | |
|---|---|
| Propin | 0,11 |
| 1,3-Butadien | 99,73 |
| Wasser | 0,16 |

sowie in einen Sumpfstrom K I-S, mit nachstehender Zusammensetzung in Gew.-%:

| | |
|---|---|
| cis-Buten-2 | 0,52 |
| 1,3-Butadien | 40,0 |
| 1,2-Butadien | 15,1 |
| 1-Butin | 13,75 |
| Vinylacetylen | 29,17 |
| 3-Methylbuten-1 | 0,98 |
| 2-Methylbuten-2 | 0,48. |

Der Kopfstrom K I-K der ersten Destillationskolonne K I wurde in einen Abzug (1/7 des Kopfstromes K I-K) und in einen Rücklauf (6/7 des Kopfstromes K I-K) aufgeteilt. Der Abzug wurde einer zweiten Destillationskolonne K II mit 25 theoretischen Trennstufen, auf die 14. Trennstufe zugeführt, und in einen Kopfstrom K II-K mit folgender Zusammensetzung in Gew.-%:

| | |
|---|---|
| Propin | 79,52 |
| 1,3-Butadien | 20,0 und |
| Wasser | 0,48 |

sowie einen Sumpfstrom K II-S, umfassend Rein-1,3-Butadien, mit einem Gehalt an 1,3-Butadien von 99,99 %, aufgetrennt. Der Sumpfstrom K II-S wurde als Wertprodukt abgezogen.

Nach der vorstehend beschriebenen erfindungsgemäßen Auftrennung eines C₄-Schnittes in einer Extraktivdestillationskolonne in einen die Butane, die Butene sowie einen Roh-1,3-Butadien umfassenden Strom, mit nachfolgenden destillativer Aufarbeitung des Roh-1,3-Butadienstromes war gegenüber einem bekannten Verfahren, mit Abtrennung von Butanen und Butenen in getrennten Verfahrensstufen, wie beispielsweise in der deutschen Patentanmeldung 102 19 375 beschrieben und mit Abtrennung der Acetylene aus dem Roh-1,3-Butadienstrom durch Extraktivdestillation mit einem selektiven Lösungsmittel, wie beispielsweise in der deutschen Patentanmeldung 101 05 660 beschrieben, der Bedarf an von außen zugeführter Energie um 15 % niedriger.

## Patentansprüche

1. Kontinuierliches Verfahren zur Auftrennung eines C₄-Schnittes (C₄) durch Extraktivdestillation mit einem selektiven Lösungsmittel (LM) in einer Extraktivdestillationskolonne (EDK), **dadurch gekennzeichnet, dass** in der Extraktivdestillationskolonne (EDK) in Längsrichtung derselben eine Trennwand (TW) unter Ausbildung eines ersten Teilbereichs (A), eines zweiten Teilbereichs (B) und eines unteren gemeinsamen Kolonnenbereiches (C) angeordnet ist, und dass aus dem ersten Teilbereich (A) ein die Butane umfassender Kopfstrom (C₄H₁₀), aus dem zweiten Teilbereich (B) ein die Butene umfassender Kopfstrom (C₄H₈) und aus dem unteren gemeinsamen Kolonnenbereich (C) ein die Kohlenwasserstoffe aus dem C₄-Schnitt umfassender Strom (C₄H₆) abgetrennt wird, die im selektiven Lösungsmittel (LM) besser löslich sind als die Butane und die Butene.

2. Verfahren nach Anspruch 1, **dadurch gekennzeichnet, dass** der die Kohlenwasserstoffe aus dem C₄-Schnitt (C₄) umfassende Strom (C₄H₆), die im selektiven Lösungsnuttel (LM) besser löslich sind als die Butane und die Butene, als Seitenstrom aus dem unteren gemeinsamen Kolonnenbereich (C) und das selektive Lösungsmittel (LM) als Sumpfstrom aus der Extraktivdestillationskolonne (EDK) abgezogen wird.

3. Verfahren nach Anspruch 1, **dadurch gekennzeichnet, dass** der die Kohlenwasserstoffe aus dem C₄-Schnitt (C₄) umfassende Strom (C₄H₆), die im selektiven Lösungsmittel (LM) besser löslich sind als die Butane und die Butene gemeinsam mit dem selektiven Lösungsmittel (LM) als Sumpfstrom aus der Extraktivdestillationskolonne (EDK) abgezogen werden.

4. Verfahren nach einem der Ansprüche 1 bis 3, **dadurch gekennzeichnet, dass** der C₄-Schnitt (C₄) dem ersten Teilbereich (A) der Extraktivdestillationskolonne (EDK) zugeführt, der die Butane umfassende Kopfstrom (C₄H₁₀) aus dem Teilbereich (A) der Extraktivdestillationskolonne (EDK) und der die Butene umfassende Kopfstrom (C₄H₈) aus dem Teilbereich (B) der Extraktivdestillationskolonne (EDK) abgezogen werden.

5. Verfahren nach einem der Ansprüche 1 bis 3, **dadurch gekennzeichnet, dass** anstelle der Extraktivdestillationskolonne (EDK) mit Trennwand (TW) zwei oder mehrere, bevorzugt zwei oder drei thermisch gekoppelte Kolonnen eingesetzt werden.

6. Verfahren nach einem der Ansprüche 1 bis 5, **dadurch gekennzeichnet, dass** als selektives Lösungsmittel eine oder mehrere der Substanzen: Dimethylformamid, Acetonitril, Furfurol, N-Methylpyrrolidon (NMP), bevorzugt NMP in wässriger Lösung, eingesetzt wird.

7. Verfahren nach einem der Ansprüche 1 bis 6, **dadurch gekennzeichnet, dass** im Bereich der Trennwand der Extraktivdestillationskolonne (EDK) 10 bis 80, bevorzugt 25 theoretische Trennstufen angeordnet sind.

8. Verfahren nach einem der Ansprüche 1 bis 7, **dadurch gekennzeichnet, dass** in der Extraktivdestillationskolonne (EDK) zusätzlich eine Selektivhydrierung in heterogener Katalyse der Dreifachbindungen aufweisenden Kohlenwasserstoffe aus dem C₄-Schnitt (C₄) zu Doppelbindungen aufweisenden Kohlenwasserstoffen durchgeführt wird.

9. Verfahren nach einem der Ansprüche 1 bis 7, **dadurch gekennzeichnet, dass** der aus der Extraktivdestillationskolonne abgezogene Strom (C₄H₆), der die im selektiven Lösungsmittel (LM) besser als die Butane und Butene löslichen Kohlenwasserstoffe umfasst, einer ersten Destillationskolonne (K I) zugeführt wird, worin er in einen Kopfstrom (K I-K) aufgetrennt wird, umfassend 1,3-Butadien, Propin, gegebenenfalls weitere Leichtsieder und gegebenenfalls Wasser sowie in einen Sumpfstrom (K I-S), umfassend 1,3-Butadien, 1,2-Butadien, Acetylene sowie gegebenenfalls weitere Schwersieder, wobei der Anteil des 1,3-Butadiens im Sumpfstrom (K I-S) der Destillationskolonne (K I) dergestalt geregelt wird, dass er mindestens so hoch ist, dass er die Acetylene außerhalb des selbstzersetzungsgefährdeten Bereiches verdünnt und dass der Kopfstrom (KI-K) der ersten Destillationskolonne (K I) in einer zweiten Destillationskolonne (K II) zugeführt und in der zweiten Destillationskolonne (K II) in einen Kopfstrom (K II-K), umfassend Propin, gegebenenfalls weitere Leichtsieder und gegebenenfalls Wasser und in einen Sumpfstrom (K II-S), umfassend Rein-1,3-Butadien, aufgetrennt wird.

10. Verfahren nach Anspruch 1, **dadurch gekennzeichnet, dass** der Sumpfstrom aus der ersten Destillationskolonne und der Kopfstrom aus der zweiten Destillationskolonne einer Reaktivdestillationskolonne zugeführt werden, in der in heterogener Katalyse mit Wasserstoff eine Selektivhydrierung der Dreifachbindungen aufweisenden Kohlenwasserstoffe zu Doppelbindungen aufweisenden Kohlenwasserstoffen durchgeführt wird, bei Teilumsatz der Acetylene, unter Erhalt eines Kopfstromes, umfassend 1,3-Butadien, Butane, Butene sowie nicht-hydrierte Kohlenwasserstoffe mit Dreifachbindungen und einen Schwersieder umfassenden Sumpfstrom, der ausgeschleust wird.

11. Verfahren zur Auftrennung eines C₄-Schnittes nach einem der Ansprüche 1. bis 8 **gekennzeichnet durch** den weiteren Verfahrensschritt, dass der die Butene Isobuten, 1-Buten und 2-Butene umfassende Strom (C₄H₈) in einer Reaktivdestillationskolonne zu einem überwiegend Isobuten umfassenden Strom und einen überwiegend 2-Butene umfassenden Strom weiterverarbeitet wird, wobei in der Reaktivdestillationskolonne 1-Buten- zu 2-Butenen hydroisomerisiert wird und der überwiegend Isobuten umfassende Strom als Kopfstrom der Reaktivdestillationskolonne und der überwiegend 2-Butene umfassende Strom als Sumpfstrom der Reaktivdestillationskolonne abgezogen wird.

12. Verfahren zur Auftrennung eines C₄-Schnittes nach einem der Ansprüche 1 bis 8 **gekennzeichnet durch** den weiteren Verfahrensschritt, dass der die Butene umfassende Strom (C₄H₈) einer selektiven Veretherung des Isobutens und Auftrennung in einen den Isobutenether umfassenden Strom und in einen 1-Buten und 2-Butene umfassenden Strom unterworfen wird und der 1-Buten und die 2-Butene umfassende Strom anschließend **durch** Gasphasenisomerisierung der 2-Butene zu einem überwiegend 1-Buten umfassenden Strom oder **durch** Hydroisomerisierung des 1-Butens zu einem überwiegend 2-Butene umfassenden Strom weiterverarbeitet wird.

13. Verfahren zur Auftrennung eines C₄-Schnittes nach einem der Ansprüche 1 bis 8 **gekennzeichnet durch** den weiteren Verfahrensschritt, dass der die Butene Isobuten, 1-Buten und 2-Butene umfassende Strom (C₄H₈) **durch** Skelettisomerisierung von 1-Buten und 2-Butenen zu Isobuten weiterverarbeitet wird, wobei ein überwiegend Isobuten umfassender Strom erhalten wird.

14. Verfahren zur Auftrennung eines C₄-Schnittes nach einem der Ansprüche 1 bis 8 **gekennzeichnet durch** den weiteren Verfahrensschritt, dass der die Butene Isobuten, 1-Buten und 2-Butene umfassende Strom (C₄H₈) weiterverarbeitet wird, indem Isobuten abgetrennt und **durch** Skelettisomerisierung ein überwiegend 1-Buten und 2-Butenen umfassender Strom erhalten wird.

15. Verfahren zur Auftrennung eines C₄-Schnittes nach einem der Ansprüche 1 bis 8 **gekennzeichnet durch** den weiteren Verfahrensschritt, dass der die Butene Isobuten, 1-Buten und 2-Butene umfassende Strom (C₄H₈) weiterverarbeitet wird, indem Isobuten abgetrennt und **durch** Hydrierung ein überwiegend Isobutan umfassender Strom erhalten wird, der vorzugsweise einem Cracker zugeführt oder durch Skelettisomerisierung zu einem überwiegend n-Butan umfassenden Strom und Dehydrierung desselben zu einem überwiegend 1-Buten und 2-Butene umfassenden Strom weiterverarbeitet wird.

16. Verfahren zur Auftrennung eines C₄-Schnittes nach einem der Ansprüche 1 bis 8 **gekennzeichnet durch** den weiteren Verfahrensschritt, dass der die Butene Isobuten, 1-Buten und 2-Butene umfassende Strom (C₄H₈) weiterverarbeitet wird, indem das Isobuten zu den entsprechenden C₈-Kohlenwasserstoffen selektiv dimerisiert wird und nachfolgend eine destillative Auftrennung in einen 1-Buten und 2-Butene umfassenden Strom und einen die C₈-Kohlenwasserstoffe umfassenden Strom, erfolgt.

## Claims

1. A continuous process for fractionating a C₄ fraction (C₄) by extractive distillation using a selective solvent (LM) in an extractive distillation column (EDK), wherein a dividing wall (TW) is installed in the longitudinal direction in the extractive distillation column (EDK) to form a first region (A), a second region (B) and a lower combined column region (C) and a top stream (C₄H₁₀) comprising the butanes is taken off from the first region (A), a top stream (C₄H₈) comprising the butenes is taken off from the second region (B) and a stream (C₄H₆) comprising the hydrocarbons from the C₄ fraction which are more soluble in the selective solvent (LM) than are the butanes and the butenes is taken off from the lower combined column region (C).

2. The process according to claim 1, wherein the stream (C₄H₆) comprising the hydrocarbons from the C₄ fraction (C₄) which are more soluble in the selective solvent (LM) than are the butanes and the butenes is taken off as a side stream from the lower combined column region (C) and the selective solvent (LM) is taken off as bottom stream from the extractive distillation column (EDK).

3. The process according to claim 1, wherein the stream (C₄H₆) comprising the hydrocarbons from the C₄ fraction (C₄) which are more soluble in the selective solvent (LM) than are the butanes and the butenes is taken off together with the selective solvent (LM) as bottom stream from the extractive distillation column (EDK).

4. The process according to any of claims 1 to 3, wherein the C₄ fraction (C₄) is fed into the first region (A) of the extractive distillation column (EDK), the top stream (C₄H₁₀) comprising the butanes is taken off from the region (A) of the extractive distillation column (EDK) and the top stream (C₄H₈) comprising the butenes is taken off from the region (B) of the extractive distillation column (EDK).

5. The process according to any of claims 1 to 3, wherein two or more, preferably two or three, thermally coupled columns are used in place of the extractive distillation column (EDK) with dividing wall (TW).

6. The process according to any of claims 1 to 5, wherein the selective solvent used comprises one or more of the substances: dimethylformamide, acetonitrile, furfural, N-methylpyrrolidone (NMP), preferably NMP in aqueous solution.

7. The process according to any of claims 1 to 6, wherein from 10-80, preferably 25, theoretical plates are located in the region of the dividing wall of the extractive distillation column (EDK).

8. The process according to any of claims 1 to 7, wherein a heterogeneously catalyzed selective hydrogenation of the hydrocarbons containing triple bonds from the C₄ fraction (C₄) to hydrocarbons containing double bonds is additionally carried out in the extractive distillation column (EDK).

9. The process according to any of claims 1 to 7, wherein the stream (C₄H₆) comprising the hydrocarbons which are more soluble in the selective solvent (LM) than are the butanes and butenes which is taken off from the extractive distillation column is fed to a first distillation column (K I) in which it is separated into a top stream (K I-K) comprising 1,3-butadiene, propyne, possibly further low boilers and possibly water, and a bottom stream (K I-S) comprising 1,3-butadiene, 1,2-butadiene, acetylenes and possibly further high boilers, with the proportion of 1,3-butadiene in the bottom stream (K I-S) from the distillation column (K I) being regulated so that it is sufficiently high to dilute the acetylenes to outside the range in which there is a risk of spontaneous decomposition and the top stream (K I-K) from the first distillation column (K I) is fed to a second distillation column (K II) and in this is separated into a top stream (K II-K) comprising propyne, possibly further low boilers and possibly water and a bottom stream (K II-S) comprising pure 1,3-butadiene.

10. The process according to claim 1, wherein the bottom stream from the first distillation column and the top stream from the second distillation column are passed to a reactive distillation column in which a heterogeneously catalyzed selective hydrogenation of the hydrocarbons containing triple bonds to hydrocarbons containing double bonds is carried out by means of hydrogen, with a partial conversion of the acetylenes, to give a top stream comprising 1,3-butadiene, butanes, butenes and non-hydrogenated hydrocarbons having triple bonds and a bottom stream comprising high boilers which is discharged.

11. The process for fractionating a C₄ fraction according to any of claims 1 to 8, which comprises the further process step of processing the stream (C₄H₈) comprising the butenes isobutene, 1-butene and 2-butenes further in a reactive distillation column to give a stream comprising predominantly isobutene and a stream comprising predominantly 2-butenes, with 1-butene being hydroisomerized to 2-butenes in the reactive distillation column and the stream comprising predominantly isobutene being taken off as top stream from the reactive distillation column and the stream comprising predominantly 2-butenes being taken off as bottom stream from the reactive distillation column.

12. The process for fractionating a C₄ fraction according to any of claims 1 to 8, which comprises the further process step of subjecting the stream (C₄H₈) comprising the butenes to a selective etherification of the isobutene and fractionation to give a stream comprising the isobutene ether and a stream comprising 1-butene and 2-butenes and subsequently processing the stream comprising 1-butene and the 2-butenes further by gas-phase isomerization of the 2-butenes to give a stream comprising predominantly 1-butene or by hydroisomerization of the 1-butene to give a stream comprising predominantly 2-butenes.

13. The process for fractionating a C₄ fraction according to any of claims 1 to 8, which comprises the further process step of processing the stream (C₄H₈) comprising the butenes isobutene, 1-butene and 2-butenes further by skeletal isomerization of 1-butene and 2-butenes to isobutene, giving a stream comprising predominantly isobutene.

14. The process for fractionating a C₄ fraction according to any of claims 1 to 8, which comprises the further process step of processing the stream (C₄H₈) comprising the butenes isobutene, 1-butene and 2-butenes further by separating off isobutene and converting it by skeletal isomerization into a stream comprising predominantly 1-butene and 2-butenes.

15. The process for fractionating a C₄ fraction according to any of claims 1 to 8, which comprises the further process step of processing the stream (C₄H₈) comprising the butenes isobutene, 1-butene and 2-butenes further by separating off isobutene and converting it by hydrogenation into a stream which comprises predominantly isobutane and is preferably fed to a cracker or processed further by skeletal isomerization to give a stream comprising predominantly n-butane or dehydrogenation of the latter to give a stream comprising predominantly 1-butene and 2-butenes.

16. The process for fractionating a C₄ fraction according to any of claims 1 to 8, which comprises the further process step of processing the stream (C₄H₈) comprising the butenes isobutene, 1-butene and 2-butenes further by selectively dimerizing the isobutene to form the corresponding C₈-hydrocarbons and subsequently carrying out a fractional distillation to give a stream comprising 1-butene and 2-butenes and a stream comprising the C₈-hydrocarbons.

## Revendications

1. Procédé en mode continu pour séparer une coupe en C₄ (C₄) par distillation extractive avec un solvant sélectif (LM) dans une colonne de distillation extractive (EDK), **caractérisé en ce que** la colonne de distillation extractive (EDK) est pourvue, dans le sens de sa longueur, d'une paroi séparatrice (TW), ce qui permet de former une première zone partielle (A), une deuxième zone partielle (B) et une zone de colonne inférieure commune (C), et **en ce que** l'on sépare de la première zone partielle (A), un courant de tête comprenant les butanes (C₄H₁₀), de la deuxième zone partielle (B), un courant de tête comprenant les butènes (C₄H₈) et de la zone de colonne inférieure commune (C), un courant comprenant les hydrocarbures de la coupe en C₄ (C₄H₆), lesquels sont plus solubles dans le solvant sélectif (LM) que les butanes et les butènes.

2. Procédé selon la revendication 1, **caractérisé en ce que** l'on extrait de la colonne de distillation extractive (EDK) le courant comprenant les hydrocarbures (C₄H₆) de la coupe en C₄ (C₄), lesquels sont plus solubles dans le solvant sélectif (LM) que les butanes et les butènes, sous la forme d'un courant latéral à partir de la zone de colonne inférieure commune (C), et le solvant sélectif (LM) sous la forme d'un courant de bas de colonne.

3. Procédé selon la revendication 1, **caractérisé en ce que** l'on extrait de la colonne de distillation extractive (EDK) le courant comprenant les hydrocarbures (C₄H₆) de la coupe en C₄ (C₄), lesquels sont plus solubles dans le solvant sélectif (LM) que les butanes et les butènes, conjointement avec le solvant sélectif (LM) sous la forme d'un courant de bas de colonne.

4. Procédé selon l'une quelconque des revendications 1 à 3, **caractérisé en ce que** la coupe en C₄ (C₄) est acheminée à la première zone partielle (A) de la colonne de distillation extractive (EDK), **en ce que** l'on extrait le courant de tête comprenant les butanes (C₄H₁₀) de la zone partielle (A) de la colonne de distillation extractive (EDK) et le courant de tête comprenant les butènes (C₄H₈) de la zone partielle (B) de la colonne de distillation extractive (EDK).

5. Procédé selon l'une quelconque des revendications 1 à 3, **caractérisé en ce que** l'on utilise, à la place de la colonne de distillation extractive (EDK) munie d'une paroi séparatrice (TW), deux ou plusieurs colonnes couplées thermiquement, de préférence, deux ou trois.

6. Procédé selon l'une quelconque des revendications 1 à 5, **caractérisé en ce que** l'on utilise comme solvant sélectif une ou plusieurs substances choisies parmi le diméthylformamide, l'acétonitrile, le furfurol, la N-méthylpyrrolidone (NMP), de préférence, la NMP en solution aqueuse.

7. Procédé selon l'une quelconque des revendications 1 à 6, **caractérisé en ce que** 10 à 80, de préférence, 25 étages de séparation théoriques sont aménagés dans la zone de la paroi séparatrice de la colonne de distillation extractive (EDK).

8. Procédé selon l'une quelconque des revendications 1 à 7, **caractérisé en ce que** l'on effectue en outre, dans la colonne de distillation extractive (EDK), une hydrogénation sélective par catalyse hétérogène des hydrocarbures présentant des triples liaisons de la coupe en C₄ (C₄), de manière à obtenir des hydrocarbures présentant des doubles liaisons.

9. Procédé selon l'une quelconque des revendications 1 à 7, **caractérisé en ce que** le courant (C₄H₆) extrait de la colonne de distillation extractive, qui comprend les hydrocarbures plus solubles dans le solvant sélectif (LM) que les butanes et les butènes, est acheminé à une première colonne de distillation (K I), dans laquelle il est séparé en un courant de tête (K I-K) comprenant du 1,3-butadiène, du propyne et, éventuellement, d'autres substances à bas point d'ébullition et, éventuellement, de l'eau, et en un courant de bas de colonne (K I-S) comprenant du 1,3-butadiène, du 1,2-butadiène, de l'acétylène ainsi que, éventuellement, d'autres substances à point d'ébullition élevé, la fraction du 1,3-butadiène étant ajustée dans le courant de bas de colonne (K I-S) de la colonne de distillation (K I), de sorte qu'elle soit au moins suffisamment élevée pour diluer les acétylènes en dehors de la plage de risques de décomposition spontanée, et **en ce que** le courant de tête (KI-K) de la première colonne de distillation (K I) est acheminé à une deuxième colonne de distillation (K II) et que celui-ci est séparé dans la deuxième colonne de distillation (K II) en un courant de tête (K II-K) comprenant du propyne, éventuellement, d'autres substances à bas point d'ébullition et, éventuellement, de l'eau, et en un courant de bas de colonne (K II-S) comprenant du 1,3-butadiène pur.

10. Procédé selon la revendication 1, **caractérisé en ce que** le courant de bas de colonne provenant de la première colonne de distillation et le courant de tête provenant de la deuxième colonne de distillation sont acheminés vers une colonne de distillation réactive, dans laquelle on effectue, par le biais d'une catalyse hétérogène avec de l'hydrogène, une hydrogénation sélective des hydrocarbures présentant des triples liaisons afin d'obtenir des hydrocarbures présentant des doubles liaisons, avec une conversion partielle des acétylènes, tout en obtenant un courant de tête comprenant du 1,3-butadiène, des butanes, des butènes, ainsi que des hydrocarbures non hydrogénés ayant des triples liaisons, et un courant de bas de colonne comprenant des substances à bas point d'ébullition, que l'on décharge.

11. Procédé de séparation d'une coupe en C₄ selon l'une quelconque des revendications 1 à 8, **caractérisé par** l'autre étape de procédé qui consiste à transformer le courant comprenant les butènes des types isobutène, 1-butène et 2-butènes (C₄H₈), dans une colonne de distillation réactive, en un courant comprenant principalement de l'isobutène et en un courant comprenant principalement des 2-butènes, dans lequel on effectue, dans la colonne de distillation réactive, l'hydroisomérisation du 1-butène en 2-butènes, et on extrait le courant comprenant principalement de l'isobutène sous la forme d'un courant de tête de la colonne de distillation réactive, et le courant comprenant principalement des 2-butènes sous la forme d'un courant de bas de colonne de la colonne de distillation réactive.

12. Procédé de séparation d'une coupe en C₄ selon l'une quelconque des revendications 1 à 8, **caractérisé par** l'autre étape de procédé qui consiste à soumettre le courant comprenant les butènes (C₄H₈) à une éthérification sélective de l'isobutène et à le séparer en un courant comprenant l'éther d'isobutène et en un courant comprenant du 1-butène et des 2-butènes, et à transformer ensuite le courant comprenant le 1-butène et les 2-butènes, par isomérisation en phase gazeuse des 2-butènes, en un courant comprenant principalement du 1-butène ou, par hydroisomérisation du 1-butène, en un courant comprenant principalement des 2-butènes.

13. Procédé de séparation d'une coupe en C₄ selon l'une quelconque des revendications 1 à 8, **caractérisé par** l'autre étape de procédé qui consiste à transformer le courant comprenant les butènes des types isobutène, 1-butène et 2-butènes (C₄H₈), par isomérisation du squelette du 1-butène et des 2-butènes, en isobutène, de manière à obtenir un courant comprenant principalement de l'isobutène.

14. Procédé de séparation d'une coupe en C₄ selon l'une quelconque des revendications 1 à 8, **caractérisé par** l'autre étape de procédé qui consiste à transformer le courant comprenant les butènes des types isobutène, 1-butène et 2-butènes (C₄H₈), dans lequel on sépare l'isobutène et on obtient, par isomérisation du squelette, un courant comprenant principalement du 1-butène et des 2-butènes.

15. Procédé de séparation d'une coupe en C₄ selon l'une quelconque des revendications 1 à 8, **caractérisé par** l'autre étape de procédé qui consiste à transformer le courant comprenant les butènes des types isobutène, 1-butène et 2-butènes (C₄H₈), dans lequel on sépare l'isobutène et on obtient, par hydrogénation, un courant comprenant principalement de l'isobutane que l'on achemine, de préférence, à un dispositif de craquage ou que l'on transforme, par isomérisation du squelette, en un courant comprenant principalement du n-butane et, en effectuant la déshydrogénation de celui-ci, en un courant comprenant principalement du 1-butène et des 2-butènes.

16. Procédé de séparation d'une coupe en C₄ selon l'une quelconque des revendications 1 à 8, **caractérisé par** l'autre étape de procédé qui consiste à transformer le courant comprenant les butènes des types isobutène, 1-butène et 2-butènes (C₄H₈), dans lequel on effectue la dimérisation sélective de l'isobutène en hydrocarbures en C₈ correspondants, et on effectue ensuite une séparation par distillation pour obtenir un courant comprenant du 1-butène et des 2-butènes et un courant comprenant les hydrocarbures en C₈.
